(19) Europäisches Patentamt
European Patent Office
Office européen des brevets

(11) EP 0 638 639 B1

(12) **EUROPEAN PATENT SPECIFICATION**

(45) Date of publication and mention of the grant of the patent:
**12.04.2000 Bulletin 2000/15**

(51) Int. Cl.[7]: **C11D 1/62**, C07C 229/12

(21) Application number: **94202175.9**

(22) Date of filing: **25.07.1994**

(54) **Biodegradable fabric softening composition**

Biologisch abbaubare Wäscheweichspülerzusammensetzung

Composition adoucissante biodégradable pour le linge

(84) Designated Contracting States:
**AT BE CH DE DK ES FR GB GR IE IT LI LU MC NL PT SE**

(30) Priority: **10.08.1993 EP 93202352**

(43) Date of publication of application:
**15.02.1995 Bulletin 1995/07**

(73) Proprietor: **Akzo Nobel N.V.**
**6824 BM Arnhem (NL)**

(72) Inventors:
- **Weissen, Hans Joachim**
**D-52372 Kreuzau (DE)**
- **Porta, Norbert**
**D-52388 Norvinich-Eggerheims (DE)**

(74) Representative:
**Schalkwijk, Pieter Cornelis et al**
**AKZO NOBEL N.V.**
**Patent Department (Dept. APTA)**
**P.O. Box 9300**
**6800 SB Arnhem (NL)**

(56) References cited:
**EP-A- 0 239 910**
**EP-A- 0 267 551**
**EP-A- 0 550 361**
**WO-A-91/16880**
**DE-A- 2 700 512**

- **DATABASE WPI Week 8439, Derwent Publications Ltd., London, GB; AN 84-240086 & JP-A-59 142 299 (AJINOMOTO KK ET AL) 15 August 1984**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

The invention relates to fabric softening compositions which contain as essential constituent a biodegradable quaternary ammonium compound. Softening compositions of the above-mentioned type have earlier been suggested in US-A-A 767 547. Preferred among the plurality of compositions described therein are those containing as essential constituent compounds of the formula N,N-di(alkanoyl-oxy-ethyl)-N,N-dimethyl ammonium chloride, their principal representative being N,N-di(tallowoyl-oxy-ethyl)-N,N-dimethyl ammonium chloride.
However, further improvement of the biodegradability of said compounds was desired, as was further improvement of their toxicity to algae and their suitability to form concentrated dispersions and/or solutions.

WO-A-91/16880 relates to liposomes containing quaternary ammonium compounds and to a process for the preparation of said liposomes. This document does not relate to the technical problem of the instant invention, i.e. the provision of a fabric softening composition containing as essential constituent a biodegradable quaternary ammonium compound. In Examples 11 and 14 two compounds are disclosed, i.e. 1,2-dipalmitoyl-sn-glycero-3-N,N,N-trimethylglycine ester chloride and 1,3-dioleoylglycero-2-N,N,N-trimethylglycine ester chloride, for which no protection is sought.

JP-A-59142299 discloses cationic surfactants comprising a long-chain fatty acid ester of a cationised polyol containing a tertiary or quaternary nitrogen substituent and their use in detergents and shampoos. These compounds bear no chemical relation to the compounds of the presently claimed composition.

The invention now provides fabric softening compositions comprising quaternary ammonium compounds which, by and large, satisfy the above-listed desires.

The invention consists in that the fabric softening compositions of the known type mentioned in the opening paragraph contain as essential constituent a quaternary ammonium compound of the formula:

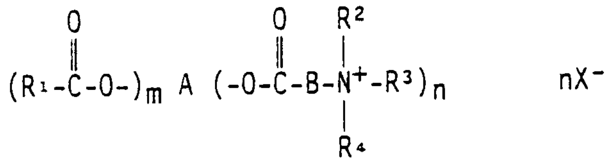

wherein X is an anion, A is an (m+n) valent radical remaining after the removal of (m+n) hydroxy groups from an aliphatic polyol, natural polyol, oligomer and/or polymer containing repeating units of vinyl alcohol having p hydroxy groups and an atomic ratio of carbon to oxygen in the range of 1,0 to 3,0 and up to 2 groups per hydroxy group selected from ethylene oxide and propylene oxide,
m is 0 or an integer from 1 to p-n, n is an integer from 1 to p-m, and
p is an integer of at least 2,
B is an alkylene or alkylidene group containing 1 to 4 carbon atoms,
$R^1$, $R^2$, $R^3$ and $R^4$ are, independently from each other, straight or branched chain $C_1$-$C_{48}$ alkyl or alkenyl groups, optionally with substitution by one or more functional groups and/or interruption by at most 10 ethylene oxide and/or propylene oxide groups or by at most two functional groups selected from

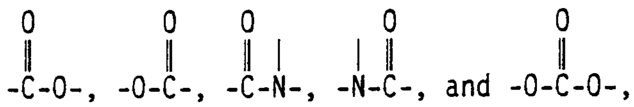
and

or $R^2$ and $R^3$ may form a ring system containing 5 or 6 atoms in the ring,
with the proviso that the average compound either has at least one R group having 22-48 carbon atoms, or at least two R groups having 16-20 carbon atoms, or at least three R groups having 10-14 carbon atoms.

Good results are generally obtained with fabric softening compositions containing as essential constituent a quaternary ammonium compound according to the first mentioned formula in which (m+n) is at least 2.

It should be noted that compounds of a similar structure have already been proposed for use in detergent compositions in US-A-4 260 529. None of the compounds specifically disclosed therein satisfies the general formula according to the present invention. It should therefore be considered extremely surprising that quaternary ammonium compounds of a structure comparable with that in said US patent specification, but derived from an aliphatic polyol natural polyol, oligomer and/or polymer containing repeating units of vinyl alcohol having p hydroxy groups, lead to compositions which not only have good softening properties but also may be applied in a relatively low viscous, highly concentrated form, without the use of any dispersing aids. Under similar circumstances the known quaternary ammonium compounds-containing compositions will become pasty or even solid or tend to gel.

The preparation of the present compositions comprising the quaternary ammonium compounds according to the first structural formula can be in the manner known for the preparation of analogous compounds. The quaternization is preferably carried out in the presence of appropriate solvents, e.g., water and/or organic solvents such as low-molecular weight alkanols, e.g., ethanol, isopropanol, diols, e.g., ethyleneglycol, diethyleneglycol, or polyols, e.g., glycerol, or in the presence of the lower alkyl ethers or esters of said compounds, e.g., ethyleneglycol monomethyl ether, diglycolbutyl ether, and/or methoxy polyethyleneglycol.

Within the scope of the invention preference is given to compositions comprising compounds corresponding to the first structural formula obtainable by reaction of a polyol having 2 to 8 hydroxy groups. Optimum results are obtained when the polyol is selected from the group of ethyleneglycol, glycerol, pentaerythritol, sorbitol, glucose, methylglucoside, and/or condensed derivatives thereof. The polyol may be partially etherified and/or esterified.

Good results can be obtained when the ester forming derivative of the polyol or the carboxylic acid is a natural fat, preferably a glyceride. Examples of condensed derivatives according to the invention are diglycerol, triglycerol, and dipentaerythritol.

Use may also be made of natural polyols such as starch, degraded starch, and oligomers and/or polymers containing repeating units of vinyl alcohol.

Compositions comprising compounds corresponding to the first structural formula are obtainable by reaction of an aliphatic carboxylic acid of the formula $R^1$-COOH or an ester thereof. Optimum results are obtained when said acid is tallow acid and/or at least partially hydrogenated tallow acid. Other examples of suitable quaternary ammonium compounds corresponding to the first structural formula are obtained by replacing tallow acid with, e.g., coconut acid, palmitic acid, lauric acid, oleic acid, stearic acid, or the like.

The $R^2$, $R^3$, and $R^4$ groups are, independently from each other, straight or branched chain $C_1$-$C_{48}$ alkyl or alkenyl groups, optionally with substitution by one or more functional groups and/or interruption by at most 10 ethylene oxide and/or propylene oxide groups, or by at most two functional groups selected from

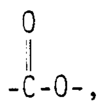, 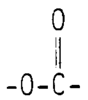, 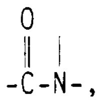, 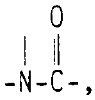, and 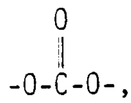,

or $R^2$ and $R^3$ may form a ring system containing 5 or 6 atoms in the ring. Typical combinations of $R^2/R^3/R^4$ are: $CH_3/CH_3/CH_3$, $CH_3/CH_3/C_{16/18}$ alk(en)yl, $CH_3/CH_3/CH_2CH_2OOCC_{15/17}$ alk(en)yl, $CH_3/CH_3/CH_2COOC_{16/18}$ alk(en)yl, $CH_3/CH_3/CH_2CH_2CH_2NHCOC_{15/17}$ alk(en)yl.

The $R^2$,$R^3$ and $R^4$ groups can be introduced into the molecule by the use of appropriate tertiary amines such as trimethylamine, (hydrogenated) tallow fatty acid ester of N,N-dimethylethanolamine, (hydrogenated) tallow alcohol ester of N,N-dimethylglycine, (hydrogenated) tallow fatty acid amide of N,N-dimethylaminopropylamine, and dimethyl (hydrogenated) tallowamine.

The $R^2$ and $R^3$ groups can also be introduced by the use of appropriate secondary amines such as dimethylamine. The $R^4$ group can then be introduced by quaternization of the thus obtained tertiary amine with halogenating agents such as methyl chloride and dimethylsulphate.

If for the preparation of compounds satisfying the first structural formula use is made of a $C_{1-4}$ halocarboxylic acid, preference is given to a monochlorocarboxylic acid, such as 2-chloropropionic acid or 3-chloropropionic acid, optimum results being obtained with monochloroacetic acid.

If use is made of a tertiary amine, preference is given to trimethylamine and dimethylethanolamine esterified with a $C_{10-22}$ carboxylic acid.

Good results may also be obtained when for the tertiary amine use is made of dimethylaminopropylamine amidated with a $C_{10-22}$ carboxylic acid.

All that can be required of the anion $X^-$ contained as counterion in the present quaternary ammonium compounds is for it to be compatible with the cationic fabric softening action, to be itself non-toxic, and to generate a water-soluble or water-dispersible salt with the quaternary ammonium cation. Preferably, X- represents a halide anion, more particularly a chloride, sulphate, methosulphate, carboxylate, or sulphonate anion.

The invention further relates to a process for preparing compounds comprising the quaternary ammonium compounds according to the first-mentioned formula, by

a) reacting in a first step 1 equivalent of an aliphatic polyol, natural polyol, oligomer and/or polymer containing

repeating units of vinyl alcohol having p free and/or esterified hydroxy groups with m equivalents of an aliphatic acid of the formula $R^1$-COOH or of an ester thereof and n equivalents of a $C_{1-4}$ halocarboxylic acid or of an ester thereof, under reduced pressure at a temperature between 50° and 150°C, followed by either

b) heating with n equivalents of a tertiary amine of the formula $R^2R^3R^4N$, optionally in an organic solvent, at a temperature between 40° and 150°C, preferably between 50° and 100°C, or

c) heating with n equivalents of a secondary amine of the formula $R^2R^3NH$, optionally in an organic solvent, at a temperature between 40° and 150°C, preferably between 50° and 100°C, followed by conversion with n equivalents of a quaternizing agent, such as methylchloride or dimethylsulphate.

The first reaction step is generally carried out in the presence of a catalyst such as p-toluene sulphonic acid under a reduced pressure of 20-30 mbar and at a temperature of about 60°C while stripping off the water, followed by an increase in the temperature up to 150°C over 2-5 hours. The polyol may be partially etherified and/or esterified. Mixtures of polyols are also suitable. If the polyol is glycerol, use may also be made of a mixture of a natural fat and glycerol or another polyol. If use is made of a mixture of a natural fat and a polyol, a preceding transesterification may be carried out under basic conditions, with use being made of a basic catalyst such as sodium methylate or sodium.

During the second reaction step the chlorinated polyol ester is mixed with the desired alkylamine, preferably in appropriate solvents, e.g., water and/or organic solvents such as low-molecular weight alkanols, e.g., ethanol, isopropanol, diols, e.g., ethyleneglycol, diethyleneglycol, or polyols, e.g., glycerol, or in the presence of the lower alkyl ethers or esters of said compounds, e.g., ethyleneglycol monomethyl ether, diglycolbutyl ether, and/or methoxy polyethyleneglycol, followed by heating for 1-2 hours at a temperature between 80° and 100°C. Another process consists in dissolving the chlorinated polyol ester in e.g. isopropanol, followed by adding trimethylamine for 10-30 minutes in an autoclave at a pressure < 5 bar at an initial temperature of about 50°C. Due to the exothermic reaction, the temperature increases to about 80°C, and the reaction mixture is maintained at said temperature for 0,5-1 hour.

A very attractive process for preparing compounds comprising the quaternary ammonium compounds according to the first formula is characterised by

a) reacting in a first step 1 equivalent of an aliphatic polyol, natural polyol, oligomer and/or polymer containing repeating units of vinyl alcohol having p esterified hydroxy groups with ≤ p moles of dimethyl aminoethanol at a temperature between 80° and 200°C in the presence of an alkaline catalyst, followed by the distillation of excess dimethyl aminoethanol,

b) esterification in a separate step of an aliphatic polyoly, natural polyol, oligomer and/or polymer containing repeating units of vinyl alcohol optionally esterified, with at least two moles of chloroacetic acid per mole of polyol, followed by

c) quaternisation of the transesterified tertiary amine obtained in the first reaction step with the esterification product of the second reaction step.

The first reaction step is generally carried out in the presence of an alkaline catalyst such as sodium methyl ate or sodium. Instead of dimethyl aminoethanol use may also be made of other tertiary amino alcohols such as methyl diethanolamine.

The aliphatic polyol preferably is glycerol, which may be wholly or partially esterified with a fatty acid.

The second reaction step is identical with step a) of the process described hereinbefore. For the aliphatic polyol, natural polyol, oligomer and/or polymer containing repeating units of vinyl alcohol optionally esterified, use may be made of the by-product of the transesterification reaction of the first step.

The quaternisation reaction of the transesterified tertiary amine obtained in the first reaction step with the chlorinated polyol ester is preferably carried out in appropriate solvents, e.g., water and/or organic solvents such as low-molecular weight alkanols, e.g., ethanol, isopropanol, diols, e.g., ethylene glycol, diethylene glycol, or polyols, e.g., glycerol, or in the presence of the lower alkyl ethers or esters of said compounds, e.g., ethylene glycol monomethyl ether, diglycol butyl ether, and/or methoxy polyethylene glycol, followed by refluxing for 1-2 hours at a temperature between 80° and 100°C.

The fabric softening compositions comprising the quaternary ammonium compounds according to the first structural formula can be used to form dispersions and/or solutions displaying a relatively low viscosity at high concentrations. Non-aqueous solutions may be formed of which 5 up to 90 wt.% of the solids content consists of the compositions according to the invention. Generally, 5 up to 60 wt.% of aqueous solutions consists of the solids content of said compositions, while in the case of aqueous dispersions 5 up to 40 wt.% may consist of the solids content of said compositions.

Tallow is a convenient and inexpensive source of long chain alkyl and alkenyl material. Specific examples of components suitable for preparing the rapidly biodegradable quaternary ammonium compounds according to the invention are given in the Tables below. The components in the first Table comprise a polyol, chloroacetic acid, and a fatty

alkyl-containing tertiary amine:

Table I

| Polyol | Amine |
|---|---|
| glycerol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| pentaerythritol | N,N-dimethyl-N-(H-tallowyl) |

| | |
|---|---|
| " | N,N-dimethyl-N-(H-tallowoyl-oxyethyl) |
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-di-hydroimidazole |
| " | 2-(H-tallowyl)-1-(H-tallowyl-amidoethyl)-4,5-di-hydroimidazole |
| diglycerol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-di-hydroimidazole |
| " | 2-(H-tallowyl)-1-(H-tallowyl-amidoethyl)-4,5-di-hydroimidazole |
| ethyleneglycol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-di-hydroimidazole |
| " | 2-(H-tallowyl)-1-(H-tallowyl-amidoethyl)-4,5-di-hydroimidazole |
| propyleneglycol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-di-hydroimidazole |
| " | 2-(H-tallowyl)-1-(H-tallowyl-amidoethyl)-4,5-di-hydroimidazole |
| glucose | N,N-dimethyl-N-(H-tallowyl) |
| " | N,N-dimethyl-N-(H-tallowoyl-oxyethyl) |

| | |
|---|---|
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| methyl-α-D-glucopyranoside | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| saccharose | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| sorbitol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |

The components in the following Table comprise a polyol esterified with hydrogenated tallow acid, chloroacetic acid, and a tertiary amine.

Table II

| Polyol | Tertiary amine |
|---|---|
| glycerol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |
| " | N,N,N-triethyl |
| " | N,N-dimethyl-N-(hydroxyethyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| pentaerythritol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |
| " | N,N,N-triethyl |
| " | N,N-dimethyl-N-(hydroxyethyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| diglycerol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |

| | |
|---|---|
| " | N,N,N-triethyl |
| " | N,N-dimethyl-N-(hydroxyethyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| ethyleneglycol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |
| " | N,N,N-triethyl |
| " | N,N-dimethyl-N-(hydroxyethyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| propyleneglycol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |
| " | N,N,N-triethyl |
| " | N,N-dimethyl-N-(hydroxyethyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| glucose | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |

| | |
|---|---|
| " | N,N,N-triethyl |
| " | N,N-dimethyl-N-(hydroxyethyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| methyl-α-D-glucopyranoside | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |
| " | N,N,N-triethyl |
| " | N,N-dimethyl-N-(hydroxyethyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| saccharose | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |
| " | N,N,N-triethyl |
| " | N,N-dimethyl-N-(hydroxyethyl) |
| " | N-methyl-N,N-di(H-tallowyl) |
| " | 2-(H-tallowyl)-1-(H-tallowoyl-oxyethyl)-4,5-dihydroimidazole |
| " | 2-(H-tallowyl)-1-(H-tallowyl-amidoethyl)-4,5-dihydroimidazole |
| sorbitol | N,N-dimethyl-N-(H-tallowyl) |
| " | N,N-dimethyl-N-(H-tallowoyl-oxyethyl) |
| " | N,N-dimethyl-N-(H-tallowyl-amidopropyl) |
| " | N,N,N-trimethyl |

" N,N,N-triethyl
" N,N-dimethyl-N-(hydroxyethyl)
" N-methyl-N,N-di(H-tallowyl)
" 2-(H-tallowyl)-1-(H-tallowoyl-oxyethyl)-4,5-dihydroimidazole
" 2-(H-tallowyl)-1-(H-tallowyl-amidoethyl)-4,5-dihydroimidazole

In the above examples the hydrogenated tallow residue may be replaced by a fatty alkyl derived from other natural sources such as tallow oil, coconut oil, palmitic oil, and rape oil, all of which may be saturated or unsaturated.

Typical fabric softening compositions according to the present invention include the following quaternary ammonium compounds:

bis (H-tallowbetaine) glycerol ester
tris (H-tallowbetaine) glycerol ester

(H-tallowbetaine) H-tallowoyl glycerol ester
bis (H-tallowbetaine) H-tallowoyl glycerol ester

betaine di-(H-tallowoyl) glycerol ester

bis (H-tallowbetaine) pentaerythritol ester
tris (H-tallowbetaine) pentaerythritol ester
tetra (H-tallowbetaine) pentaerythritol ester

(H-tallowbetaine) H-tallowoyl pentaerythritol ester
bis (H-tallowbetaine) H-tallowoyl pentaerythritol ester
tris (H-tallowbetaine) H-tallowoyl pentaerythritol ester

betaine di-(H-tallowoyl) pentaerythritol ester

bis (H-tallowbetaine) diglycerol ester
tris (H-tallowbetaine) diglycerol ester
tetra (H-tallowbetaine) diglycerol ester

(H-tallowbetaine) H-tallowoyl diglycerol ester
bis (H-tallowbetaine) H-tallowoyl diglycerol ester
tris (H-tallowbetaine) H-tallowoyl diglycerol ester

betaine di-(H-tallowoyl) diglycerol ester

bis (H-tallowbetaine) ethyleneglycol ester

(H-tallowbetaine) H-tallowoyl ethyleneglycol ester

bis (H-tallowbetaine) propyleneglycol ester

(H-tallowbetaine) H-tallowoyl propyleneglycol ester

bis (H-tallowbetaine) glucose ester
tris (H-tallowbetaine) glucose ester

tetra (H-tallowbetaine) glucose ester

(H-tallowbetaine) H-tallowoyl glucose ester
bis (H-tallowbetaine) H-tallowoyl glucose ester
tris (H-tallowbetaine) H-tallowoyl glucose ester

betaine di-(H-tallowoyl) glucose ester

bis (H-tallowbetaine) methyl-α-D-glucopyranoside ester
tris (H-tallowbetaine) methyl-α-D-glucopyranoside ester
tetra (H-tallowbetaine) methyl-α-D-glucopyranoside ester

(H-tallowbetaine) H-tallowoyl methyl-α-D-glucopyranoside ester
bis (H-tallowbetaine) H-tallowoyl methyl-α-D-glucopyranoside ester
tris (H-tallowbetaine) H-tallowoyl methyl-α-D-glucopyranoside ester

betaine di-(H-tallowoyl) methyl-α-D-glucopyranoside ester

bis (H-tallowbetaine) saccharose ester
tris (H-tallowbetaine) saccharose ester
tetra (H-tallowbetaine) saccharose ester

(H-tallowbetaine) H-tallowoyl saccharose ester
bis (H-tallowbetaine) H-tallowoyl saccharose ester
tris (H-tallowbetaine) H-tallowoyl saccharose ester

betaine di-(H-tallowoyl) saccharose ester

bis (H-tallowbetaine) sorbitol ester

tris (H-tallowbetaine) sorbitol ester
tetra (H-tallowbetaine) sorbitol ester

(H-tallowbetaine) H-tallowoyl sorbitol ester
bis (H-tallowbetaine) H-tallowoyl sorbitol ester
tris (H-tallowbetaine) H-tallowoyl sorbitol ester

betaine di-(H-tallowoyl) sorbitol ester

Preferred compounds of this group are:

bis (H-tallowbetaine) glycerol ester
(H-tallowbetaine) H-tallowoyl glycerol ester
betaine di-(H-tallowoyl) glycerol ester

bis (H-tallowbetaine) pentaerythritol ester
(H-tallowbetaine) H-tallowoyl pentaerythritol ester
betaine di-(H-tallowoyl) pentaerythritol ester

bis (H-tallowbetaine) diglycerol ester
(H-tallowbetaine) H-tallowoyl diglycerol ester
betaine di-(H-tallowoyl) diglycerol ester

bis (H-tallowbetaine) ethyleneglycol ester
(H-tallowbetaine) H-tallowoyl ethyleneglycol ester

bis (H-tallowbetaine) propyleneglycol ester
(H-tallowbetaine) H-tallowoyl propyleneglycol ester

bis (H-tallowbetaine) glucose ester
(H-tallowbetaine) H-tallowoyl glucose ester
betaine di-(H-tallowoyl) glucose ester

bis (H-tallowbetaine) methyl-α-D-glucopyranoside ester
(H-tallowbetaine) H-tallowoyl methyl-α-D-glucopyranoside ester
betaine di-(H-tallowoyl) methyl-α-D-glucopyranoside ester

bis (H-tallowbetaine) saccharose ester
(H-tallowbetaine) H-tallowoyl saccharose ester
betaine di-(H-tallowoyl) saccharose ester

bis (H-tallowbetaine) sorbitol ester
(H-tallowbetaine) H-tallowoyl sorbitol ester
betaine di-(H-tallowoyl) sorbitol ester

The compositions optionally contain non-ionics. Such non-ionics comprise polyethyleneglycol, polypropyleneglycol, polyethylene/polypropylene-copolymers, ethoxylated and/or propoxylated lower alkyl amines or polyamines, ethoxylated and/or propoxylated fatty acids, partially esterified polyols, fatty acid alkanolamides, fatty alcohols or fatty amines.

The stability of the compositions can be improved further, and their viscosity adjusted, by the incorporation of small amounts of electrolyte. Examples of suitable electrolytes are the salts of lithium, sodium, potassium, magnesium chloride, calcium chloride, or aluminium chloride and quaternary (lower alkyl) ammonium salts.
Optionally, the compositions according to the invention may also comprise acids. Suitable acids are hydrochloric acid, boric acid, and phosphoric acid, or organic acids, such as glycolic acid, citric acid, and tartaric acid.

If the compositions are wholly or partially in the form of dispersions, they may optionally contain dispersion stabilisers. Such stabilisers include ethoxylated fatty alcohols and amines, preferably $C_{10-20}$ fatty alcohols ethoxylated with 5 to 100 moles of ethylene oxide (e.g., ELFAPUR T250® (talloweth-25) commercially available from Akzo Chemicals) and $C_{10-20}$ fatty amines ethoxylated with 5 to 100 moles of ethylene oxide (e.g., ETHOMEEN®HT60 (PEG-50 H-tallow amine) commercially available from Akzo Chemicals).

The compositions may optionally contain other ingredients known to be suitable for use in textile softeners. Such adjuvants include perfumes, preservatives, germicides, colourants, dyes, fungicides, brighteners, and opacifiers. These adjuvants, if used, are normally added at their conventional levels. However, in the case of composition ingredients utilised for a fabric treatment effect, e.g., perfumes, these materials can be added at higher than normal levels, corresponding to the degree of concentration of the product.

The invention will be further described in the following examples, which must not be construed as limiting the scope of the present invention.

The manner in which the present biodegradable fabric softening agent is applied is the same as for the conventional, now commercially available fabric softeners, where the agent, together with the surfactant, can be contacted with the textile material to be softened in the form of a rinsing agent or else in the dryer. The fabric softening action envisaged and actualised here is the same as that of the conventional fabric softeners. Inevitably, via the rinsing solution a portion of the fabric softener will end up in the waste water, where, because of the present invention, there will be less harm to the environment as a result of accelerated biodegradability.

Description of the test methods used:

1. Softening

The present quaternary ammonium compounds were tested for their fabric softening performance in a panel test. In the test prewashed terry towels treated with the fabric softeners were line dried for 24 hours and then cut up into strips of 10*20 $cm^2$. A test panel evaluated the softening action as compared with that of three standards, whereupon the observational data was statistically processed in accordance with DIN standard 10954.

Standards :

1 : dimethyl-di-(H-tallow) ammonium chloride (ARQUAD®2HT) (commercially available from Akzo Chemicals)

2 : 1-methyl-2-tallowalkyl-3-tallowamido-ethyl imidazolinium methosulphate (REWOQUAT®7500)

3 : WMP - test detergent

The ranking of the standards is:

ARQUAD®R2HT > REWOQUAT®7500 >> WMP-test detergent

2. Biodegradation

The present quaternary ammonium compounds were tested for biodegradability in accordance with the EEC/OECD guidelines OECD 301D "closed bottle test". In this experiment a test compound is added to an aqueous solution of mineral salts and exposed for 28 days under aerobic conditions to a relatively small number of micro-organisms. The formal test regulations were departed from on the following minor issues :

- the inoculum was taken from an apparatus containing activated sludge preconditioned in accordance with proposed amendments to the EEC guidelines.
- ammonium chloride was not included in the medium to avoid nitrification.

The dissolved oxygen concentrations were determined electrochemically using an oxygen electrode (WTW Trioxmatic EO 200) and an oxygen gauge (WTW OXI 530), and biodegradability was calculated as the ratio of biological oxygen demand (BOD) to theoretical oxygen demand (ThOD) :

Biodegradability = BOD/ThOD

3. Algae toxicity

The present quaternary ammonium compounds were tested for chronic algae toxicity in accordance with the EEC/OECD guidelines OECD 201 "Alga growth inhibition test". Exponentially-growing cultures of selected green algae (Selenastrum capricornutum ATCC 22662) are exposed to various concentrations of the test substance over several generations under defined conditions. The inhibition of growth in relation to a control culture is determined over a fixed period.

4. Viscosity

Brookfield LVTD, 60 rpm, 20°C, spindle 1.4.

<u>Example 1</u>

Preparation of (H-tallow betaine) glycerol esterquats

1 mole of glycerol was esterified with 2 moles of chloroacetic acid at a temperature not exceeding 130°C. The resulting glycerol ester was treated with 2 moles of N,N-dimethyl-N-(H-tallow) amine (ARMEEN®DMHTD) in isopropanol under reflux for 2 hours. The resulting glycerol esterquat was a mixture of the mono-, di-, and triquaternary ammonium compounds.

Properties:

The glycerol esterquat was soluble in water. Surprisingly, it was found that the softening performance was equal to that of REWOQUAT® 7500 despite its solubility in water.
A 5% clear, low viscous solution in water could be formed.

<u>Example 2</u>

Preparation of (H-tallow betaine) H-tallowoyl glycerol esterquats

1 mole of glycerol was esterified simultaneously with 1 mole of hydrogenated tallow fatty acid and 1,5 moles of chloroacetic acid at a temperature not exceeding 130°C. The resulting glycerol ester was treated with 1,5 moles of N,N-dimethyl-N-(H-tallow)amine (ARMEEN®DMHTD) in isopropanol under reflux for 2 hours. The resulting glycerol

esterquat was a mixture of non-ionic glycerol esters and mono-, di-, and triquaternary ammonium compounds.

When the resulting esterquat was dispersed in water, it surprisingly was found that a highly concentrated dispersion of up to 25 % by weight could be prepared without making use of any adjuvants.

Softening performance : ARQUAD®2HT = Example 2 > REWOQUAT ®W7500 >> WMP - test detergent

Surprisingly, it was found that despite the content of non-ionics, the softening performance of this esterquat was comparable with that of ARQUAD®2HT and superior to that of REWOQUAT®W7500.

Example 3

Preparation of H-tallowoyl pentaerythritol betaine esterquats

1 mole of pentaerythritol was esterified simultaneously with 2 moles of hydrogenated tallow fatty acid and 1,5 moles of chloroacetic acid at a temperature not exceeding 150°C. The resulting pentaerythritol ester was treated with 1,5 moles of trimethylamine in isopropanol in an autoclave at 80°C and a pressure of 2 bar for 2 hours. The resulting pentaerythritol esterquat was a mixture of non-ionic pentaerythritol esters and mono-, di-, tris- and tetra- quaternary ammonium compounds.

A highly concentrated dispersion of up to 40 weight% active could be made in water without the use of any adjuvants.

| | |
|---|---|
| Softening performance | ARQUAD®2HT > Example 3 > REWOQUAT®7500 >> WMP - test detergent |
| Biodegradation | 77 % (28 days) |
| Algae toxicity | > 100 ppm |
| Viscosity of dispersions | 15 mPa.s, 20 % active without additives |
| | 60 mPa.s, 30 % active without additives |
| | 1400 mPa.s, 40 % active without additives |

Example 4

Preparation of bis(H-tallow betaine) ethyleneglycol esterquat

1 mole of ethyleneglycol was esterified with 2 moles of chloroacetic acid at a temperature not exceeding 130°C. The resulting ethyleneglycol ester was then treated with 2 moles of N,N-dimethyl-N-(H-tallow) amine (ARMEEN®DMHTD) in isopropanol under reflux for 2 hours.

The softening performance of this esterquat was found to be the same as that of REWOQUAT®W7500.
The product could be dissolved in water, resulting in a clear, viscous aqueous solution containing up to 30% of active material.
A 50% active formulation could be made in a 1:1 solution of propylene glycol and isopropanol. The formulation could be dispersed rapidly in cold water without gel particles which might block the dosing compartment of the washing machine being formed.

Example 5

Preparation of bis(H-tallowoyl oxyethyl betaine) glycerol esterquat

1 mole of glycerol was esterified with 2 moles of chloroacetic acid at a temperature not exceeding 130°C. The resulting glycerol ester was treated with 2 moles of N,N-dimethyl-N-(H-tallowoyl-oxyethyl) amine in isopropanol under reflux for 2 hours.

The softening performance of this esterquat was as follows:

ARQUAD®2HT ≤ Example 5 ≤ REWOQUAT ®W7500 >> WMP - test detergent

Algae toxicity : 50 ppm

The product could be dissolved in water, resulting in a clear, viscous aqueous solution containing up to 30% of active material. A 50% active formulation could be made in a 1:1 solution of propylene glycol and isopropanol. The formulation could be dispersed rapidly in cold water without gel particles which might block the dosing compartment of the washing machine being formed.

Example 6

Preparation of bis(H-tallowoyl oxyethyl betaine) sorbitol esterquat

1 mole of sorbitol was esterified with 2,1 moles of chloroacetic acid at a temperature not exceeding 130°C. The resulting sorbitol ester was treated with 2,1 moles of N,N-dimethyl-N-(H-tallowoyl-oxyethyl) amine in isopropanol under reflux for 2 hours.

The resulting reaction product displayed the following properties: Softening performance:

ARQUAD® 2HT > Example 6 = REWOQUAT® 7500 >> WMP-test detergent

Algae toxicity : 50 ppm

The product could be dissolved in water, resulting in a clear, slightly viscous aqueous solution containing up to 30% of active material.
A 50% active formulation could be made in a 1:1 solution of propylene glycol and isopropanol. The formulation could be dispersed rapidly in cold water without gel particles which might block the dosing compartment of the washing machine being formed.

Example 7

Preparation of (RA-oyl-oxyethyl betaine) glycerol esterquat

1 mole of glycerol was esterified simultaneously with 2 moles of chloroacetic acid at a temperature not exceeding 130°C. The resulting glycerol ester was subsequently heated under reflux in isopropanol for 2 hours with 2 moles of a product obtained by the transesterification of 1 mole of rapeseed oil and 20 moles of dimethyl aminoethanol (DMAE) for 30 minutes at 140°C in the presence of 0,5 wt.% KOH as catalyst, followed by the distillation of excess DMAE.

The resulting esterquat could be dissolved in water, resulting in a clear, viscous aqueous solution containing up to 30% of active material.
A clear, viscous, 50% active formulation could be made in a 1:1 solution of isopropanol and ethylene glycol/propylene glycol (EG/PG).

Example 8

Preparation of bis(tallowoyl oxyethyl betaine) glycerol esterquats

1 mole of glycerol was esterified with 2 moles of chloroacetic acid at a temperature not exceeding 130°C. The resulting glycerol ester was heated under reflux in isopropanol with 2 moles of N,N-dimethyl-N ((partially hydrogenated) tallowoyl-oxyethyl) amine (tallow fatty acid of IV 50) at 80°C for 2 hours.
The resulting quaternary reaction mixture is liquid at room temperature (cloud point (4°C/h): 11°C, non pour point (4°C/h): 10°C). The resulting esterquat containing 16% isopropanol was mixed with propylene glycol (60% isopropanol containing esterquat/ 40% propylene glycol). The cloud point of this composition was 4°C, the non-pour point 3°C.
The composition could easily be diluted with water without using any stirring equipment.

Example 9

Preparation of bis(tallowoyl oxyethyl betaine) ethylene glycol esterquat

1 mole of ethylene glycol was esterified with 2 moles of chloroacetic acid at a temperature not exceeding 130°C. The resulting ethylene glycol ester was then heated with 2 moles of N,N-dimethyl-N-(tallowoyl-oxyethyl) amine

(tallow fatty acid of IV 50) in isopropanol under reflux for 2 hours.

The resulting water-soluble quaternary ammonium compound was well-suited to prepare highly concentrated liquid formulations with low cloud points and low non-pour points.

The resulting quaternary reaction mixture containing 17% isopropanol was mixed with propylene glycol (60% isopropanol containing esterquat/ 40% propylene glycol). The cloud point of this composition was 4°C, the non pour point (cooling rate 4°C/h) 2°C.

The product could easily be diluted with water even when the water was cold and agitation was poor.

**Claims**

1. A fabric softening composition containing as essential constituent a biodegradable quaternary ammonium compound, characterised in that the composition comprises at least one or more compounds of the formula:

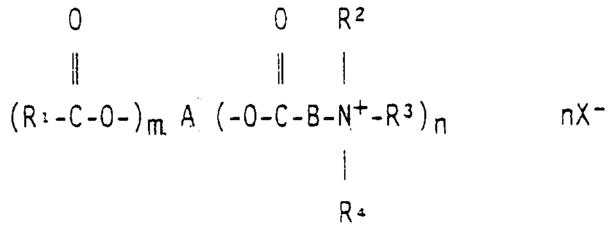

wherein X is an anion, A is an (m+n) valent radical remaining after the removal of (m+n) hydroxy groups from an aliphatic polyol, natural polyol, oligomer and/or polymer containing repeating units of vinyl alcohol having p hydroxy groups and an atomic ratio of carbon to oxygen in the range of 1,0 to 3,0 and up to 2 groups per hydroxy group selected from ethylene oxide and propylene oxide,

m is 0 or an integer from 1 to p-n, n is an integer from 1 to p-m, and p is an integer of at least 2,

B is an alkylene or alkylidene group containing 1 to 4 carbon atoms,

$R^1$, $R^2$, $R^3$ and $R^4$ are, independently from each other, straight or branched chain $C_1$-$C_{48}$ alkyl or alkenyl groups, optionally with substitution by one or more functional groups and/or interruption by at most 10 ethylene oxide and/or propylene oxide groups, or by at most two functional groups selected from

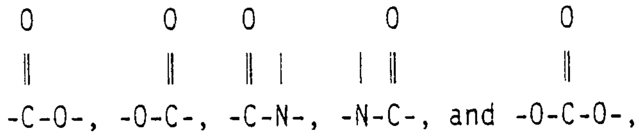

or $R^2$ and $R^3$ may form a ring system containing 5 or 6 atoms in the ring, with the exception of 1,2-dipalmitoyl-sn-glycero-3-N,N,N-trimethylglycine ester chloride and 1,3-dioleoyl-glycero-2-N,N,N-trimethylglycine ester chloride,

with the proviso that the average compound either has at least one A group having 22-48 carbon atoms, or at least two R groups having 16-20 carbon atoms, or at least three R groups having 10-14 carbon atoms.

2. A composition according to claim 1, characterised in that (m+n) is at least 2.

3. A composition according to claim 1, characterised in that the polyol contains 2 to 8 hydroxy groups.

4. A composition according to claim 3, characterised in that the polyol is selected from the group of ethylene glycol , glycerol pentaerythritol, sorbitol, glucose, saccharose, methyl glucoside, and/or condensed derivatives thereof.

5. A composition according to claim 3, characterised in that the polyol is selected from the group of diglycerol, triglycerol, and dipentaerythritol.

6. A composition according to claim 1, characterised in that the polyol is selected from the group of natural polyols

such as starch, degraded starch, and oligomers and/or polymers containing repeating units of vinyl alcohol.

**7.** A composition according to claim 1, characterised in that $R^1$-COO# is derived from tallow acid and/or at least partially hydrogenated tallow acid.

**8.** A composition according to claim 1, characterised in that B is a methylene group.

**9.** A composition according to claim 1, characterised in that $R^2$, $R^3$, and $R^4$ are methylene groups.

**10.** A composition according to claim 1, characterised in that $R^2$ and $R^3$ are methyl groups and $R^4$ is a $C_{10-22}$ carboxyethyl group.

**11.** A composition according to claim 1, characterised in that $R^2$ and $R^3$ are methyl groups and $R^4$ is a $C_{10-22}$ carbamidopropyl group.

**12.** A composition according to claim 1, characterised in that $(R^1\text{-COO-})_mA$ is a natural fat, preferably a glyceride.

**13.** A non-aqueous solution of which 5 up to 90 wt.% of the solids content consists of the composition of claim 3.

**14.** An aqueous solution of which 5 up to 60 wt.% of the solids content consists of the composition of claim 3.

**15.** An aqueous dispersion of which 5 up to 40 wt.% of the solids content consists of the composition of claim 3.

**16.** A process for preparing a composition comprising quaternary ammonium compounds according to claim 1, characterised by

a) reacting in a first step 1 equivalent of an aliphatic polyol, natural polyol, oligomer and/or polymer containing repeating units of vinyl alcohol having p free and/or esterified hydroxy groups with m equivalents of an aliphatic acid of the formula $R^1$-COOH or of an ester thereof and n equivalents of a $C_{1-4}$ halocarboxylic acid or of an ester thereof, under reduced pressure at a temperature between 50° and 150°C, followed by either
b) heating with n equivalents of a tertiary amine of the formula $R^2R^3R^4N$, optionally in an organic solvent, at a temperature between 40° and 150°C, preferably between 50° and 100°C, or
c) heating with n equivalents of a secondary amine of the formula $R^2R^3NH$, optionally in an organic solvent, at a temperature between 40° and 150°C, preferably between 50° and 100°C, followed by conversion with n equivalents of a quaternizing agent, such as methylchloride or dimethylsulphate.

**17.** A process for preparing a composition comprising quaternary ammonium compounds according to claim 1, characterised by

a) reacting in a first step 1 equivalent of an aliphatic polyol, natural polyol, oligomer and/or polymer containing repeating units of vinyl alcohol having p esterified hydroxy groups with $\leq$ p moles of dimethyl aminoethanol at a temperature between 80° and 200°C in the presence of an alkaline catalyst, followed by `the distillation of excess dimethyl aminoethanol,
b) esterification in a separate step of an aliphatic polyol, natural polyol, oligomer and/or polymer containing repeating units of vinyl alcohol optionally esterified with at least two moles of chloroacetic acid per mole of polyol, followed by
c) quaternisation of the transesterified tertiary amine obtained in the first reaction step with the esterification product of the second reaction step.

**18.** Process according to claim 17, characterised in that the optionally esterified polyol used in the second reaction step is a by-product of the transesterification reaction.

**19.** Process according to claim 17, characterised in that for the aliphatic polyol use is made of glycerol.

**20.** A quaternary ammonium compound selected from the group consisting of bis (H-tallowbetaine) glycerol ester, tris (H-tallowbetaine) glycerol ester, (H-tallowbetaine) H-tallowoyl glycerol ester, bis (H-tallowbetaine) H-tallowoyl-glycerol ester, betaine di-(H-tallowoyl) glycerol ester, bis (H-tallowbetaine) pentaerythritol ester, tris (H-tallowbetaine) pentaerythritol ester, tetra (H-tallowbetaine) pentaerythritol ester, (H-tallowbetaine) H-tallowoyl pentaerythritol

ester, bis (H-tallowbetaine) H-tallowoyl pentaerythritol ester, tris (H-tallowbetaine) H-tallowoyl pentaerythritol ester, betaine di-(H-tallowoyl) pentaerythritol ester, bis (H-tallowbetaine) diglycerol ester, tris (H-tallowbetaine) diglycerol ester, tetra (H-tallowbetaine) diglycerol ester, (H-tallowbetaine) H-tallowoyl diglycerol ester, bis (H-tallowbetaine) H-tallowoyl diglycerol ester, tris (H-tallowbetaine) H-tallowoyl diglycerol ester, betaine di-(H-tallowoyl) diglycerol ester, bis (H-tallowbetaine) ethyleneglycol ester, (H-tallowbetaine) H-tallowoyl ethyleneglycol ester, bis (H-tallowbetaine) propyleneglycol ester, (H-tallowbetaine) H-tallowoyl propyleneglycol ester, bis (H-tallowbetaine) glucose ester, tris (H-tallowbetaine) glucose ester, tetra (H-tallowbetaine) glucose ester, (H-tallowbetaine) H-tallowoyl glucose ester, bis (N-tallowbetaine) H-tallowoyl glucose ester, tris (H-tallowbetaine) H-tallowoyl glucose ester, betaine di-(H-tallowoyl) glucose ester, bis (H-tallowbetaine) methyl-α-D-glucopyranoside ester, tris (H-tallowbetaine) methyl-α-D-glucopyranoside ester, tetra (H-tallowbetaine) methyl-α-D-glucopyranoside ester, (H-tallowbetaine) H-tallowoyl methyl-α-D-glucopyranoside ester, bis (H-tallowbetaine) H-
tallowoyl methyl-α-D-glucopyranoside ester, tris (H-tallowbetaine) H-tallowoyl methyl-α-D-glucopyranoside ester, betaine di-(H-tallowoyl) methyl-α-D-glucopyranoside ester, bis (H-tallowbetaine) saccharose ester, tris (H-tallowbetaine) saccharose ester, tetra (H-tallowbetaine) saccharose ester, (H-tallowbetaine) H-tallowoyl saccharose ester, bis (H-tallowbetaine) H-tallowoyl saccharose ester, tris (H-tallowbetaine) H-tallowoyl saccharose ester, betaine di-(H-tallowoyl) saccharose ester, bis (H-tallowbetaine) sorbitol ester, tris (H-tallowbetaine) sorbitol ester, tetra (H-tallowbetaine) sorbitol ester, (H-tallowbetaine) H-tallowoyl sorbitol ester, bis (H-tallowbetaine) H-tallowoyl sorbitol ester, tris (H-tallowbetaine) H-tallowoyl sorbitol ester, and betaine di-(H-tallowoyl) sorbitol ester.

**21.** A quaternary ammonium compound according to claim 20, characterized in that the compound is selected from the group consisting of bis (H-tallowbetaine) glycerol ester, (H-tallowbetaine) H-tallowoyl glycerol ester, betaine di-(H-tallowoyl) glycerol ester, bis (H-tallowbetaine) pentaerythritol ester, (H-tallowbetaine) H-tallowoyl pentaerythritol ester, betaine di-(H-tallowoyl) pentaerythritol ester, bis (H-tallowbetaine) diglycerol ester, (H-tallowbetaine) H-tallowoyl diglycerol ester, betaine di-(H-tallowoyl) diglycerol ester, bis (H-tallowbetaine) ethyleneglycol ester, (H-tallowbetaine) H-tallowoyl ethyleneglycol ester, bis (H-tallowbetaine) propyleneglycol ester, (H-tallowbetaine) H-tallowoyl propyleneglycol ester, bis (H-tallowbetaine) glucose ester, (H-tallowbetaine) H-tallowoyl glucose ester, betaine di-(H-tallowoyl) glucose ester, bis (H-tallowbetaine) methyl-α-D-glucopyranoside ester, (H-tallowbetaine) H-tallowoyl methyl-α-D-glucopyranoside ester, betaine di-(H-tallowoyl) methyl-α-D-glucopyranoside ester, bis (H-tallowbetaine) saccharose ester, (H-tallowbetaine) H-tallowoyl saccharose ester, betaine di-(H-tallowoyl) saccharose ester, bis (H-tallowbetaine) sorbitol ester, (H-tallowbetaine) H-tallowoyl sorbitol ester, and betaine di-(H-tallowoyl) sorbitol ester.

## Patentansprüche

**1.** Textilweichmacherzusammensetzung, die als Wesentliche Komponente eine biologisch abbaubare quaternäre AmmoniumVerbindung enthält, dadurch gekennzeichnet, dass der Weichmacher mindestens eine oder mehrere Verbindungen der Formel

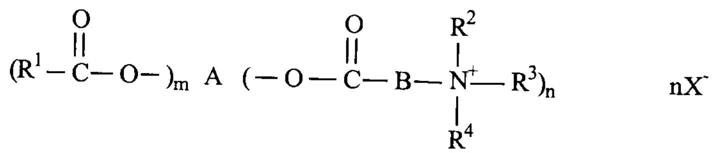

enthält, worin X ein Anion, A ein (m+n) Wertiger Rest ist, der Zurückbleibt nach der Entfernung von (m+n) Hydroxylgruppen aus einem aliphatischen Polyol, natürlichen Polyol, Oligomer und/oder Wiederkehrende Vinylalkoholeinheiten enthaltenden Polymer mit p-Hydroxygruppen und einem Atomverhältnis von Kohlenstoff zu Sauerstoff im Bereich von 1,0 bis 3,0 und bis zu zwei Gruppen pro Hydroxygruppe gewählt aus Ethylenoxid und Propylenoxid,
m null oder eine Ganzzahl von 1 bis p-n ist, n eine Ganzzahl von 1 bis p-m und p eine Ganzzahl von mindestens 2 ist,
B eine Alkylen- oder Alkylidengruppe enthaltend 1 bis 4 Kohlenstoffatome ist,
$R^1$, $R^2$, $R^3$ und $R^4$ unabhängig voneinander geradkettige oder verzweigte $C_1$ bis $C_{48}$-Alkyl- oder Alkenylgruppen, gegebenenfalls mit Substitution durch eine oder mehrere funktionelle Gruppen und/oder Unterbrechung

durch höchstens zehn Ethylenoxid- und/oder Propylenoxidgruppen oder durch mindestens zwei funktionelle Gruppen gewählt aus

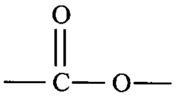, 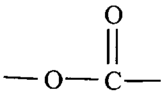, 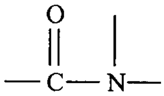, 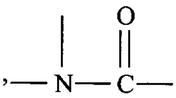, und 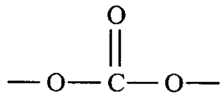

oder wobei $R^2$ und $R^3$ ein Ringsystem bilden können, das 5 oder 6 Atome im Ring enthält, mit der Ausnahme von 1,2-Dipalmitoyl-sn-glycero-3-N,N,N-trimethylglycinesterchlorid und 1,3-Dioleoyl-glycero-2-N,N,N-trimethylglycinesterchlorid,
mit der Massgabe, dass die durchschnittliche Verbindung entweder mindestens eine R-Gruppe mit 22 bis 48 Kohlenstoffatomen oder mindestens zwei R-Gruppen mit 16 bis 20 Kohlenstoffatomen oder mindestens drei R-Gruppen mit 10 bis 14 Kohlenstoffatomen enthält.

**2.** Zusammensetzung nach Anspruch 1, dadurch gekennzeichnet, dass (m+n) mindestens 2 beträgt.

**3.** Zusammensetzung nach Anspruch 1, dadurch gekennzeichnet, dass das Polyol 2 bis 8 Hydroxygruppen enthält.

**4.** Zusammensetzung nach Anspruch 3, dadurch gekennzeichnet, dass das Polyol gewählt ist aus der Gruppe Ethylenglycol, Glycerin, Pentaerythrit, Sorbit, Glucose, Saccharose, Methylglucosid und/oder kondensierten Derivaten hiervon.

**5.** Zusammensetzung nach Anspruch 3, dadurch gekennzeichnet, dass das Polyol gewählt ist aus der Gruppe Diglycerin, Triglycerin und Dipentaerythrit.

**6.** Zusammensetzung nach Anspruch 1, dadurch gekennzeichnet, dass das Polyol gewählt ist aus der Gruppe der natürlichen Polyole, wie Stärke und abgebaute Stärke, sowie der Oligomeren und/oder wiederkehrende Vinylalkoholgruppen enthaltenden Polymeren.

**7.** Zusammensetzung nach Anspruch 1, dadurch gekennzeichnet, dass $R^1$-COO von Talgfettsäure und/oder mindestens teilweise hydrierter Talgfettsäure abgeleitet ist.

**8.** Zusammensetzung nach Anspruch 1, dadurch gekennzeichnet, dass B eine Methylengruppe ist.

**9.** Zusammensetzung nach Anspruch 1, dadurch gekennzeichnet, dass $R^2$, $R^3$ und $R^4$ Methylgruppen sind.

**10.** Zusammensetzung nach Anspruch 1, dadurch gekennzeichnet, dass $R^2$ und $R^3$ Methylgruppen sind und $R^4$ eine $C_{10-22}$-Carboxyethylgruppe ist.

**11.** Zusammensetzung nach Anspruch 1, dadurch gekennzeichnet, dass $R^2$ und $R^3$ Methylgruppen sind und $R^4$ eine $C_{10-22}$-Carbamidopropylgruppe ist.

**12.** Zusammensetzung nach Anspruch 1, dadurch gekennzeichnet, dass $(R^1\text{-COO})_mA$ ein natürliches Fett, vorzugsweise ein Glycerid, ist.

**13.** Nichtwässrige Lösung, in der 5 bis 90 Gew.% der Feststoffanteile aus der Zusammensetzung von Anspruch 3 bestehen.

**14.** Wässrige Lösung, in der 5 bis 60 Gew.% der Feststoffanteile aus der Zusammensetzung von Anspruch 3 bestehen.

**15.** Wässrige Dispersion, in der 5 bis 40 Gew.% der Feststoffanteile aus der Zusammensetzung von Anspruch 3 bestehen.

**16.** Verfahren zur Herstellung einer Zusammensetzung, die eine quaternäre Ammoniumverbindung gemäss Anspruch

1 enthält, gekennzeichnet durch

a) Umsetzung in einem ersten Schritt von 1 Äquivalent eines p freie und/oder veresterte Hydroxygruppen enthaltenden aliphatischen Polyols, natürlichen Polyols, Oligomers und/oder wiederkehrende Vinylalkoholeinheiten enthaltenden Polymers mit m Äquivalenten einer aliphatischen Säure der Formel $R^1$-COOH oder einem Ester hiervon und n Äquivalenten einer $C_{1-4}$ Halogencarbonsäure oder einem Ester hiervon unter vermindertem Druck bei einer Temperatur zwischen 50° und 150°C, gefolgt von entweder
b) Erhitzen mit n Äquivalenten eines tertiären Amins der Formel $R^2R^3R^4$-N, gegebenenfalls in einem organischen Lösungsmittel, bei einer Temperatur zwischen 40° und 150°C, vorzugsweise zwischen 50° und 100°C, oder
c) Erhitzen mit n Äquivalenten eines sekundären Amins der Formel $R^2R^3$NH, gegebenenfalls in einem organischen Lösungsmittel, bei einer Temperatur zwischen 40° und 150°C, vorzugsweise zwischen 50° und 100°C, gefolgt von der Umwandlung mit n Äquivalenten eines Quaternisierungsmittel, wie Methylchlorid oder Dimethylsulfat.

**17.** Verfahren zur Herstellung einer Zusammensetzung, die quaternäre Ammoniumverbindungen gemäss Anspruch 1 enthält, gekennzeichnet durch

a) Umsetzung in einem ersten Schritt von 1 Äquivalent eines p veresterte Hydroxygruppen enthaltenden aliphatischen Polyols, natürlichen Polyols, Oligomeren und/oder wiederkehrende Vinylalkoholeinheiten enthaltenden Polymeren mit ≤ p Molen Dimethylaminoethanol bei einer Temperatur zwischen 80° und 200°C in Gegenwart eines alkalischen Katalysators, gefolgt von der Destillation von überschüssigem Dimethyaminoethanol,
b) getrenntes Verestern eines gegebenenfalls mit mindestens 2 Mol Chloressigsäure pro Mol Polyol veresterten aliphatischen Polyols, natürlichen Polyols, Oligomeren und/oder wiederkehrende Vinylalkoholeinheiten enthaltenden Polymeren, gefolgt von
c) Quaternisieren des umgeesterten tertiären Amins, das in der ersten Reaktionsstufe erhalten ist, mit dem Verresterungsprodukt der zweiten Reaktionsstufe.

**18.** Verfahren nach Anspruch 17, dadurch gekennzeichnet, dass das in der zweiten Umsetzungsstufe verwendete, gegebenenfalls veresterte Polyol ein Nebenprodukt der Umesterungsreaktion ist.

**19.** Verfahren nach Anspruch 17, dadurch gekennzeichnet, dass als aliphatisches Polyol Glycerin verwendet wird.

**20.** Quaternäre Ammoniumverbindung, gewählt aus der Gruppe bestehend aus Bis(H-talgbetain)-glycerinester, Tris(H-talgbetain)-glycerinester, (H-Talgbetain)-H-talgfettsäureglycerinester, Bis(H-talgbetain)-H-talgfettsäureglycerinester, Betain-di-(H-talgfettsäure)-glycerinester, Bis(H-talgbetain)-pentaerythritester, Tris(H-talgbetain)-pentaerythritester, Tetra-(H-talgbetain)-pentaerythritester, (H-Talgbetain)-H-talgfettsäurepentaerythritester, Bis(H-talgbetain)-H-talgfettsäurepentaerythritester, Tris(H-talgbetain)-H-talgfettsäurepentaerythritester, Betain-di-(H-talgfettsäure)-pentaerythritester, Bis(H-talgbetain)-diglycerinester, Tris(H-talgbetain)-diglycerinester, Tetra(H-talgbetain)-diglycerinester, (H-Talgbetain)-H-talgfettsäurediglycerinester, Bis(H-talgbetain)-H-talgfettsäurediglycerinester, Tris(H-talgbetain)-H-talgfettsäurediglycerinester, Betain-di-(H-talgfettsäure)-diglycerinester, Bis(H-talgbetain)-ethylengylcolester, (H-Talgbetain)-H-talgfettsäureethylenglycolester, Bis(H-talgbetain)-propylengylcolester, (H-Talgbetain)-H-talgfettsäurepropylenglycolester, Bis(H-talgbetain)-glucoseester, Tris(H-talgbetain)-glucoseester, Tetra(H-talgbetain)-glucoseester, (H-Talgbetain)-H-talgfettsäureglucoseester, Bis(H-talgbetain)-H-talgfettsäureglucoseester, Tris(H-talgbetain)-H-talgfettsäureglucoseester, Betain-di-(H-talgfettsäure)-glucoseester, Bis(H-talgbetain)-methyl-α-D-glucopyranosidester, Tris(H-talgbetain)-methyl-α-D-glucopyranosidester, Tetra(H-talgbetain)-methyl-α-D-glucopyranosidester, (H-Talgbetain)-H-talgfettsäuremethyl-α-D-glucopyranosidester, Bis(H-talgbetain)-H-talgalkylmethyl-α-D-glucopyranosidester, Tris(H-talgbetain)-H-talgfettsäuremethyl-α-D-glucopyranosidester, Betain-di-(H-talgfettsäure)-methyl-α-D-glucopyranosidester, Bis(H-talgbetain)-saccharoseester, Tris(H-talgbetain)-saccharoseester, Tetra(H-talgbetain)-saccharoseester, (H-Talgbetain)-H-talgfettsäuresaccharoseester, BiS(H-talgbetain)-H-talgfettsäuresaccharoseester, TriS(H-talgbetain)-H-talgfettsäuresaccharoseester, Betaindi-(H-talgfettsäure)-saccharoseester, Bis(H-talgbetain)-sorbitester, Tris(H-talgbetain)-sorbitester, Tetra(H-talgbetain)-sorbitester, (H-Talgbetain)-H-talgfettsäuresorbitester, Bis(H-Talgbetain)-H-talgfettsäuresorbitester, Tris(H-talgbetain)-H-talgfettsäure-sorbitester und Betain-di-(H-talgfettsäure)-sorbitester.

**21.** Quaternäre Ammoniumverbindung gemäss Anspruch 20, dadurch gekennzeichnet, dass die Verbindung gewählt ist aus der Gruppe bestehend aus Bis(H-talgbetain)-glycerinester, (H-Talgbetain)-H-talgfettsäuregylcerinester,

Betain-di-(H-Talgfettsäure)-glycerinester, Bis(H-talgbetain)-pentaerythritester, (H-Talgbetain)-H-talgfettsäurepentaerythritester, Betain-di-(H-talgfettsäure)-pentaerythritester, Bis(H-talgbetain)-diglycerinester, (H-Talgbetain)-H-talgfettsäurediglycerinester, Betain-di-(H-talgfettsäure)diglycerinester, Bis(H-talgbetain)-ethylengycolester, (H-Talgbetain)-H-talgfettsäure-ethylenglycolester, Bis(H-talgbetain)-propylenglycolester, (H-Talgbetain)-H-talgfettsäurepropylenglycolester, Bis(H-talgbetain)-glucoseester, (H-Talgbetain)-H-talgfettsäureglucoseester, Betain-di-(H-talgfettsäure)-glucoseester, Bis(H-talgbetain)-methyl-α-D-glucopyranosidester, (H-Talgbetain)-H-talgfettsäuremethyl-α-D-glucopyranosidester, Betain-di-(H-talgfettsäure)-methyl-α-D-glucopyranosidester, Bis(H-talgbetain)-saccharoseester, (H-Talgbetain)-H-talgfettsäuresaccharoseester, Betain-di-(H-talgfettsäure)-saccharoseester, Bis(H-talgbetain)-sorbitester, (H-Talgbetain)-H-talgfettsäuresorbitester und Betain-di-(H-talgfettsäure)-sorbitester.

## Revendications

**1.** Composition adoucissante pour le linge contenant comme constituant essentiel, un composé ammonium quaternaire biodégradable, caractérisée en ce que la composition comprend au moins un ou plusieurs composés ayant la formule:

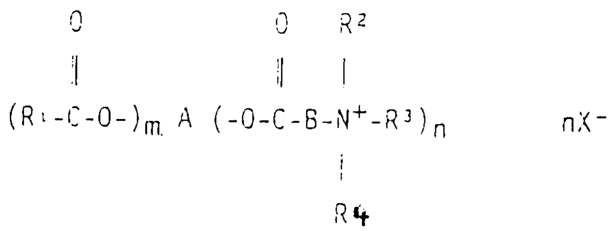

dans laquelle:

| | |
|---|---|
| X | est un anion. |
| A | est un radical (m + n)valent restant après l'élimination de (m + n) groupes hydroxy à partir d'un polyol aliphatique, d'un polyol naturel, d'un oligomère et/ou d'un polymère contenant des motifs répétés d'alcool vinylique ayant p groupes hydroxy et un rapport atomique de carbone à oxygène compris dans la gamme de 1,0 à 3,0 et jusqu'à 2 groupes par groupe hydroxy choisis parmi l'oxyde d'éthylène et l'oxyde de propylène, |
| m | est 0 ou un entier de 1 à p-n, |
| n | est un entier de 1 à p-m, et |
| p | est un entier d'au moins 2, |
| B | est un groupe alkylène ou alkylidène contenant 1 à 4 atomes de carbone, |
| $R^1$, $R^2$, $R^3$ et $R^4$ | sont indépendamment les uns des autres, des groupes alkyle ou alcényle en $C_{1-48}$ à chaîne droite ou ramifiée, éventuellement substitués par un ou plusieurs groupes fonctionnels et/ou interrompus par un maximum de 10 groupes oxyde d'éthylène et/ou oxyde de propylène ou par un maximum de deux groupes fonctionnels choisis parmi |

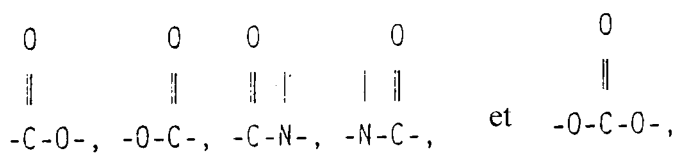

ou bien $R^2$ et $R^3$ peuvent former un système cyclique contenant 5 ou 6 atomes dans le cycle, à l'exception du chlorure d'ester de 1,2-dipalmitoyl-sn-glycéro-3-N,N,N-tri-méthylglycine et du chlorure d'ester de 1,3-dioléoyl-glycéro-2-N,N,N-triméthylglycine,

à condition que le composé moyen ait soit au moins un groupe R ayant 22 à 48 atomes de carbone, soit au moins deux groupes R ayant 16 à 20 atomes de carbone, soit au moins trois groupes R ayant 10 à 14 atomes de carbone.

**2.** Une composition suivant la revendication 1, caractérisée en ce que (m+n) est au moins 2.

3. Une composition suivant la revendication 1, caractérisée en ce que le polyol contient 2 à 8 groupes hydroxy.

4. Une composition suivant la revendication 3, caractérisée en ce que le polyol est choisi dans le groupe comprenant l'éthylène glycol, le glycérol, le pentaérythritol, le sorbitol, le glucose, le saccharose, le méthyl glucoside, et/ou des dérivés condensés de ceux-ci.

5. Une composition suivant la revendication 3, caractérisée en ce que le polyol est choisi dans le groupe comprenant le diglycérol, le triglycérol et le dipentaérythritol.

6. Une composition suivant la revendication 1, caractérisée en ce que le polyol est choisi dans le groupe des polyols naturels tels qu'un amidon, un amidon dégradé et des oligomères et/ou des polymères contenant des motifs répétés d'alcool vinylique.

7. Une composition suivant la revendication 1, caractérisée en ce que $R^1$-COOH dérive d'acide de suif et/ou d'acide de suif au moins partiellement hydrogéné.

8. Une composition suivant la revendication 1, caractérisée en ce que B est un groupe méthylène.

9. Une composition suivant la revendication 1, caractérisée en ce que $R^2$, $R^3$ et $R^4$ sont des groupes méthyle.

10. Une composition suivant la revendication 1, caractérisée en ce que $R^2$ et $R^3$ sont des groupes méthyle et $R^4$ est un groupe carboxyéthyle en $C_{10-22}$.

11. Une composition suivant la revendication 1, caractérisée en ce que $R^2$ et $R^3$ sont des groupes méthyle et $R^4$ est un groupe carbamidopropyle en $C_{10-22}$.

12. Une composition suivant la revendication 1, caractérisée en ce que $(R^1\text{-COO-})_mA$ est une graisse naturelle, de préférence un glycéride.

13. Une solution non aqueuse dont 5% jusqu'à 90% en poids de la teneur en solides consistent en la composition de la revendication 3.

14. Une solution aqueuse dont 5% jusqu'à 60% en poids de la teneur en solides consistent en la composition de la revendication 3.

15. Une dispersion aqueuse dont 5% jusqu'à 40% en poids de la teneur en solides consistent en la composition de la revendication 3.

16. Un procédé de préparation d'une composition comprenant des composés ammonium quaternaire suivant la revendication 1, caractérisé par:

a) la réaction dans une première étape d'un équivalent d'un polyol aliphatique, d'un polyol naturel d'un oligomère et/ou d'un polymère contenant des motifs répétés d'alcool vinylique ayant p groupes hydroxy libres et/ou estérifiés, avec m équivalents d'un acide aliphatique de formule $R^1$-COOH ou d'un ester de celui-ci et n équivalents d'un acide halocarboxylique en $C_{1-4}$ d'un ester de celui-ci, sous pression réduite à une température comprise entre 50°C et 150°C, suivie, soit de
b) un chauffage avec n équivalents d'une amine tertiaire de formule $R^2R^3R^4N$, éventuellement dans un solvant organique, à une température comprise entre 40°C et 150°C, de préférence entre 50°C et 100°C, soit de
c) un chauffage avec n équivalents d'une amine secondaire de formule $R^2R^3NH$, éventuellement dans un solvant organique, à une température comprise entre 40°C et 150°C, de préférence entre 50°C et 100°C, suivi d'une conversion avec n équivalents d'un agent quaternisant, comme le sulfate de diméthyle ou le chlorure de méthyle.

17. Un procédé de préparation d'une composition comprenant des composés ammonium quaternaire suivant la revendication 1, caractérisé par

a) la réaction dans une première étape d'un équivalent d'un polyol aliphatique, d'un polyol naturel, d'un oligomère et/ou d'un polymère contenant des motifs répétés d'alcool vinylique ayant p groupes hydroxy estérifiés,

avec une quantité supérieure ou égale à p moles de diméthyl aminoéthanol à une température comprise entre 80°C et 200°C en présence d'un catalyseur alcalin, suivie d'une distillation du diméthyl aminoéthanol en excès,

b) l'estérification dans une étape séparée d'un polyol aliphatique, d'un polyol naturel, d'un oligomère et/ou d'un polymère contenant des motifs répétés d'alcool vinylique, éventuellement estérifié, avec au moins deux moles d'acide chloroacétique par mole de polyol, suivie de

c) la quaternisation de l'amine tertiaire transestérifiée obtenue dans la première étape de réaction avec le produit d'estérification de la deuxième étape de réaction.

**18.** Un procédé suivant la revendication 17, caractérisé en ce que le polyol éventuellement estérifié utilisé dans la deuxième étape de réaction est un sousproduit de la réaction de transestérification.

**19.** Un procédé suivant la revendication 17 caractérisé en ce que le polyol aliphatique utilisé est le glycérol.

**20.** Un composé ammonium quaternaire choisi dans le groupe consistant en bis (H-suif-bétaïne) glycérol ester

tris (H-suif-bétaïne) glycérol ester
(H-suif-bétaïne) H-suif-oyl glycérol ester
bis (H-suif-bétaïne) H-suif-oyl glycérol ester
bétaïne-di-(H-suif-oyl) glycérol ester
bis (H-suif-bétaïne) pentaérythritol ester
tris (H-suif-bétaïne) pentaérythritol ester
tétra (H-suif-bétaïne) pentaérythritol ester
(H-suif-bétaïne) H-suif-oyl pentaérythritol ester
bis (H-suif-bétaïne) H-suif-oyl pentaérythritol ester
tris (H-suif-bétaïne) H-suif-oyl pentaérythritol ester
bétaïne di-(H-suif-oyl) pentaérythritol ester
bis (H-suif-bétaïne) diglycérol ester
tris (H-suif-bétaïne) diglycérol ester
tétra (H-suif-bétaïne) diglycérol ester
(H-suif-bétaïne) H-suif-oyl diglycérol ester
bis (H-suif-bétaïne) H-suif-oyl diglycérol ester
tris (H-suif-bétaïne) H-suif-oyl digiycerol ester
bétaïne di-(H-suif-oyl) diglycérol ester
bis (H-suif-bétaïne) éthylène glycol ester
(H-suif-bétaïne) H-suif-oyl éthylène glycol ester
bis (H-suif-bétaïne) propylène glycol ester
(H-suif-bétaïne) H-suif-oyl propylène glycol ester
bis (H-suif-bétaïne) glucose ester
tris (H-suif-bétaïne) glucose ester
tétra (H-suif-bétaïne) glucose ester
(H-suif-bétaïne) H-suif-oyl glucose ester
bis (H-suif-bétaïne) H-suif-oyl glucose ester
tris (H-suif-bétaïne) H-suif-oyl glucose ester
bétaïne di-(H-suif-oyl) glucose ester
bis (H-suif-bétaïne) méthyl-$\alpha$-D-glucopyranoside ester
tris (H-suif-bétaïne) méthyl-$\alpha$-D-glucopyranoside ester
tétra (H-suif-bétaïne) méthyl-$\alpha$-D-glucopyranoside ester
(H-suif-bétaïne) H-suif-oyl méthyl-$\alpha$-D-glucopyranoside ester
bis (H-suif-bétaïne) H-suif-oyl méthyl-$\alpha$-D-glucopyranoside ester
tris (H-suif-bétaïne) H-suif-oyl méthyl-$\alpha$-D-glucopyranoside ester
bétaïne di-(H-suif-oyl) méthyl-$\alpha$-D-glucopyranoside ester
bis (H-suif-bétaïne) saccharose ester
tris (H-suif-bétaïne) saccharose ester
tétra (H-suif-bétaïne) saccharose ester
(H-suif-bétaïne) H-suif-oyl saccharose ester
bis (H-suif-bétaïne) H-suif-oyl saccharose ester
tris (H-suif-bétaïne) H-suif-oyl saccharose ester
bétaïne di-(H-suif-oyl) saccharose ester

bis (H-suif-bétaïne) sorbitol ester
tris (H-suif-bétaïne) sorbitol ester
tétra (H-suif-bétaïne) sorbitol ester
(H-suif-bétaïne) H-suif-oyl sorbitol ester
bis (H-suif-bétaïne) H-suif-oyl sorbitol ester
tris (H-suif-bétaïne) H-suif-oyl sorbitol ester et
bétaïne di-(H-suif-oyl) sorbitol ester.

**21.** Un composé ammonium quaternaire suivant la revendication 20, caractérisé en ce que le composé est choisi dans le groupe consistant en:

bis (H-suifbétaïne) glycérol ester
(H-suif-bétaïne) H-suif-oyl glycérol ester
bétaïne di-(H-suif-oyl) glycérol ester
bis (H-suif-bétaïne) pentaérythritol ester
(H-suif-bétaïne) H-suif-oyl pentaérythritol ester
bétaïne di-(H-suif-oyl) pentaérythritol ester
bis (H-suif-bétaïne) diglycérol ester
(H-suif-bétaïne) H-suif-oyl diglycérol ester
bétaïne di-(H-suif-oyl) diglycérol ester
bis (H-suif-bétaïne) éthylène glycol ester
(H-suif-bétaïne) H-suif-oyl éthylène glycol ester
bis (H-suif-bétaïne) propylène glycol ester
(H-suif-bétaïne) H-suif-oyl propylène glycol ester
bis (H-suif-bétaïne) glucose ester
(H-suif-bétaïne) H-suif-oyl glucose ester
bétaïne di-(H-suif-oyl) glucose ester
bis (H-suif-bétaïne) méthyl-α-D-glucopyranoside ester
(H-suif-bétaïne) H-suif-oyl méthyl-α-D-glucopyranoside ester
bétaïne di-(H-suif-oyl) méthyl-α-D-glucopyranoside ester
bis (H-suif-bétaïne) saccharose ester
(H-suif-bétaïne) H-suif-oyl saccharose ester
bétaïne di-(H-suif-oyl) saccharose ester
bis (H-suif-bétaïne) sorbitol ester
(H-suif-bétaïne) H-suif-oyl sorbitol ester et
bétaïne di-(H-suif-oyl) sorbitol ester.